# EUROPEAN PATENT SPECIFICATION

(11) **EP 2 223 938 B1**
(45) Date of publication and mention of the grant of the patent: **10.07.2013**
(21) Application number: 10001237.6
(22) Date of filing: 08.05.2002
(51) Int. Cl.: C07K 14/575, A61K 38/22, A61P 3/10

(54) **Derivatives of exendin**
Exendinderivate
Dérivés d'exendine

(30) Priority: 10.05.2001 CN 01112856
(43) Date of publication of application: 01.09.2010
(62) Divisional of application: 02727167.5
(73) Proprietor: Shanghai Huayi Bio-Lab Co., Ltd., Hongqiao Cun, Zhoupu Pudong New Area Shanghai 201321 (CN)
(72) Inventor: Sun, Yukun, Shanghai 200233 (CN); Wu, Dengxi, Shanghai 200233 (CN); Zhu, Zhiyong, Shanghai 200233 (CN); Yu, Gang, Shanghai 200233 (CN); Shen, Chunjuan, Shanghai 200233 (CN); Zhao, Shaoling, Shanghai 200233 (CN); Zhou, Jiaxiang, Shanghai 200233 (CN)
(74) Representative: Ziebig, Marlene

(56) References cited:
- WO-A-99/25728
- WO-A1-00/69911
- WO-A1-01/04156
- ENG J ET AL: "ISOLATION AND CHARACTERIZATION OF EXENDIN-4, AN EXENDIN-3 ANALOGUE, FROM HELODERMA SUSPECTUM VENOM FURTHER EVIDENCE FOR AN EXENDIN RECEPTOR ON DISPERSED ACINI FROM GUINEA PIG PANCREAS" JOURNAL OF BIOLOGICAL CHEMISTRY, AMERICAN SOCIETY OF BIOLOGICAL CHEMISTS, BALTIMORE, MD, US, vol. 267, no. 11, 15 April 1992 (1992-04-15), pages 7402-7405, XP001084287 ISSN: 0021-9258

## Description

### Field of the Invention

The present invention relates to active peptide compounds useful for the treatment of type II diabetes. In particular, the present invention relates to derivates of an insulinotropic peptide exendin-4.

### Background of the Invention

It has been found that, with the same quantity, oral administration of glucose may stimulate more insulin secretion than injection. After many researches exploring for the reason, it has been verified that incretins play important roles in this phenomenon.

Incretins are a group of peptide compounds, among which gastric inhibitory peptide (GIP) which has strong inhibitive activity and glucagon-like peptide (GLP-1) were disclosed in 1985. There exist two kinds of GLP-1, one is GLP-1 (7-36) amide which is composed of 30 amino acid residues; the other is GLP-1(7-37) which is composed of 31 amino acid residues. The two have similar activities in stimulating insulin secretion. When acted on pancreatic islet cells in vitro. GLP-1 with a concentration as low as 1 × 10⁻¹⁰∼1×10⁻¹¹mol/L is capable of stimulating insulin secretion. It has been found that, by the analysis of DNA and amino acid sequences, these insulinotropic peptides are originated from proteolysis of a glucagons precursor "proglucagon" under the effect of an intestinal proteolytic enzyme, and therefore are called glucagon like peptides.

One characteristic of GLP-1 is that it may stimulate pancreatic beta cells to synthesize proinsulin mRNA and proinsulin, and to release insulin. The effects of GLP-1 on pancreatic beta cells depend on the concentration of glucose. When the concentration of blood glucose is higher than 6mmol/L, GLP-1 stimulates the secretion of insulin significantly; once the concentration is restored to normal level, the stimulating activity of GLP-1 cannot be observed anymore. Such a characteristic is of great use in the treatment of type II diabetes. In recent years clinical trials among type II diabetes patients also verified the effects of GLP-1 in stimulating insulin secretion and in decreasing glucose concentration.

The chemical structure of GLP-1 (7-36) amide is shown as follows:

In which:

His stands for histidine, Ala for alanine, Glu for glutamic acid, Gly for glycine, Thr for threonine, Phe for phenylalanine, Ser for serine, Asp for aspartic acid. Val for valine, Tyr for tyrosine, Leu for leucine, Gln for glutamine, Lys for lysine, Ile for isoleucine and Arg for arginine.

International application WO 91/11457 disclosed analogs of GLP-1 peptides 7-34, 7-35, 7-36, and 7-37, which were useful for treatment of type II diabetes.

A peptide compound Exendin-4 which was separated from venom of a Mexico beaded lizard (Heloderma horridum), was disclosed by Jean-Pierre Ranfman in Regulatory Peptides in 1996. Exendin-4 is composed of 39 amino acid residues with the carboxy terminus amidated. The chemical structure of Exendin-4 is shown as below:

In which:

Met stands for methionine, Asn for asparagines, and Pro for praline.

Exendin-4 shares 53% homology with GLP-1 in their amino acid sequences. Moreover, Exendin-4 has the capability to combine with a GLP-1 receptor and to stimulate insulin secretion. Therefore, it is regarded as an insulinotropic peptide.

US patent No. 05424286 disclosed results of some comparative experiments conducted between Exendin-4 and GLP-1 with respect to their stimulating effects on insulin secretion. It had been shown that, as compared to GLP-1, exendin-4 had a stronger capability to stimulate insulin secretion, and needed a lower concentration for showing such stimulating activity. Furthermore, Exendin-4 had a longer half life in vivo than that of GLP-1. In view of these characteristics, Exendin-4 may become a desirable therapeutic for treating type II diabetes.

Though Exendin-4 may be prepared by chemical synthesis methods, such as by solid phase synthesis on a peptide synthesizer, the high cost of such method and the consequential selling price keep exendin-4 from commercialization. Thus, for the purpose of commercialization, bio-engineering techniques have been tried to reduce the production cost.

### Aims of the Invention

The present invention directs to provide some derivates of an insulinotropic peptide exendin-4.Such derivates help to stimulate insulin secretion and to decrease blood glucose concentration. Therefore, they can be used for the treatment of type II diabetes. Such derivates may be prepared by synthetic chemical means, and more easily, by the recombinant techniques, which makes it possible to reduce the production cost.

### Summary of the Invention

The present invention provides derivates of an insulinotropic peptide and pharmaceutically acceptable salts produced thereof as defined in the claims. Said insulinotropic peptide has the amino acid sequence shown as below:

In which:
X represents Leu; Y represents Lys; Z represents Arg-OH.

The insulinotropic peptide derivates have the amino acid sequences as shown in Sequence Listing, in particular, as described in <210>1- <210>4.

The insulinotropic peptide derivates of this invention are amphoteric compounds, and may be sufficiently acidic or sufficiently basic to react with any of a number of inorganic bases, and inorganic and organic acids, to form a salt. Acids commonly employed to form acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenyl-sulfonic acid, carbonic acid, succinic acid, citric acid, benzoic acid, acetic acid, and the like. Examples of such salts include the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycolate, tartrate, methanesulfonate, propanesulfonate, naphthalene-1-sulfonate, naphthalene-2-sulfonate, mandelate, and the like. Preferred acid addition salts are those formed with mineral acids such as hydrochloric acid and hydrobromic acid, and, especially, hydrochloric acid.

Alkalis may also be employed to react with derivates of this invention to form salts. Representative examples of such alkalis include ammonium, alkali metals, alkali metal hydroxides, carbonates and bicarbonates. Typically, such an alkali may be sodium hydroxide, potassium hydroxide, ammonium hydroxide and potassium carbonate.

One aspect of this invention provides a method to produce an insulinotropic peptide derivate by solid phase synthesis, comprising using HMP resin as a solid phase carrier, protecting alpha-amine of an amino acid with 9-fluorenyl methoxycarbonyl (Fmoc), synthesizing a residue on a peptide synthesizer by the amino acid sequence of an insulinotropic peptide derivate, and obtaining products after separation, purification and lyophilize steps.

In another aspect, this invention provides a method to produce an insulinotropic peptide derivate by recombinant techniques, comprising:
a. synthesizing gene fragments by the amino acid sequence of an insulinotropic peptide derivate,
b. obtaining a cloned bacteria strain after ligation of the gene fragments, construction of a recombinant plasmid, culture of bacterial cells and transformation of the recombinant plasmid,
c. extracting inclusion bodys after fermentation of the bacteria strain and collapse of cell walls.
d. obtaining final product after lysis of inclusion bodys, separation of rough product, purification by HPLC and lyophilize step.

In still another aspect, this invention provides uses of such insulinotropic peptide derivates and pharmaceutically acceptable salts thereof in the treatment of type II diabetes.

This invention provides novel derivates of the peptide compound Exendin-4, which broadens the range of Exendin-4 derivates. The derivates of this invention have biological activities of stimulating insulin secretion and decreasing blood glucose concentration. They may be used for the treatment of type II diabetes mellitus. Such derivates may be prepared by chemical synthesis, and more easily, by recombinant techniques, which makes it possible to decrease the production cost. Moreover, Biological activities of such derivates have been proved by pharmacodynamic studies among non-obese diabetic (NOD) mice, when 0.1µg derivates of this invention were abdominally injected into the mice, the insulintropic peptide derivates showed obvious effects in increasing insulin secretion and decreasing blood glucose level.

### Description of the Figures

The present invention will be further illustrated with reference to the following figures and examples.

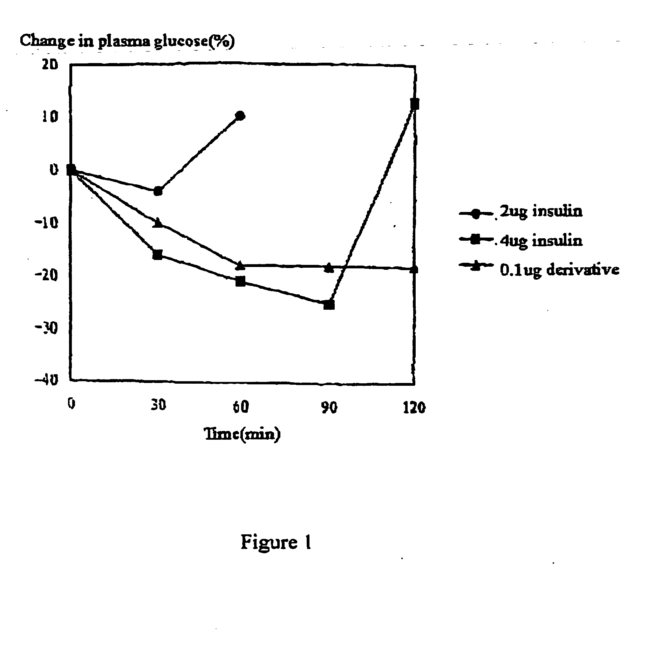

Figure 1 shows the results of pharmacodynamic studies for insulinotropic peptide derivates.

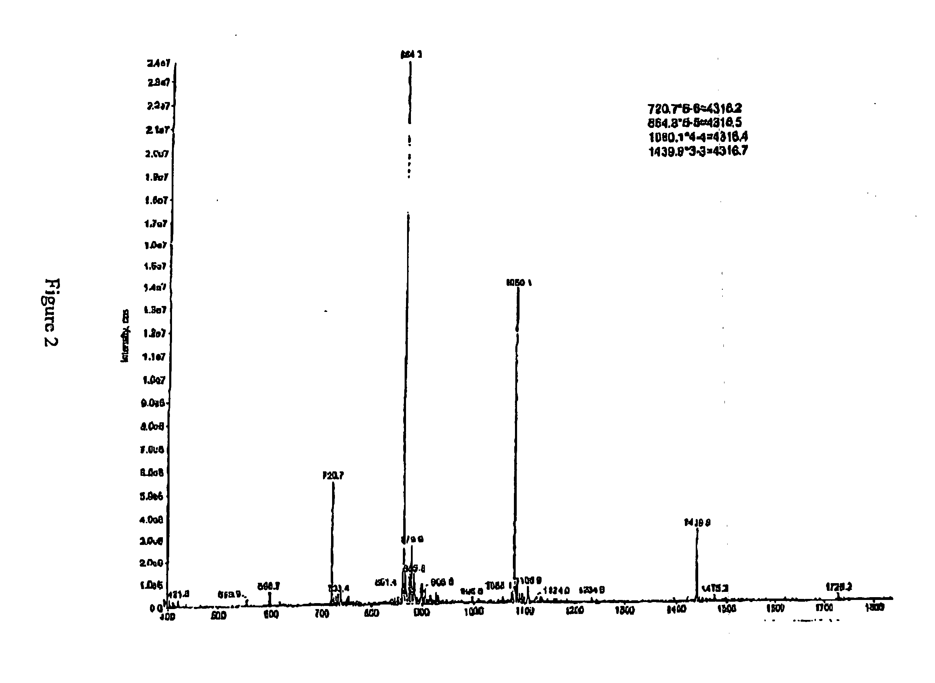

Figure 2 shows the results of mass spectrum analysis for the product prepared in Example 1, with its theoretical molecular weight 4300.6 and its determined molecular weight 4316.7.
Figure 3 schematically depicts the experiment results of decreasing blood glucose effects of Exendin 4(Lys₂₀, Arg₄₀) and Exendin 4(Leu₁₄, Lys₂₀, Arg₄₀).
Figure 4 schematically depicts the experiment results of stimulating effect of Exendin 4(Lys_{20,} Arg₄₀) on insulin-secretion.
Figure 5 schematically depicts the experiment results of C-peptide-secretion stimulating effect on for Exendin 4(Lys₂₀, Arg₄₀).

### Description of the Invention

### Example 1

### Preparation of an insulinotropic peptide derivate not according to the present invention wherein X is Arg, Y is Lys and Z is -OH by solid phase synthesis

### (1) Amino acid monomers:

| | |
|---|---|
| Fmoc-L-Ala-OH | Fmoc-L-Lys(Boc)-OH |
| Fmoc-L-Asn(Trt)-OH | Fmoc-L-Met-OH |
| Fmoc-L-Asp(OtBu)-OH | Fmoc-L-Phe-OH |
| Fmoc-L-Gln(Trt)-OH | Fmoc-L-Pro-OH |
| Fmoc-L-Glu(OtBu)-OH | Fmoc-L-Ser(tBu)-OH |
| Fmoc-L-Gly-OH | Fmoc-L-Thr(tBu)-OH |
| Fmoc-L-His(Trt)-OH | Fmoc-L-Trp-OH |
| Fmoc-L-Ile-OH | Fmoc-L-Tyr(tBu)-OH |
| Fmoc-L-Leu-OH | Fmoc-L-Val-OH |

In which:
Fmoc stands for 9-fluorenyl methoxycarbonyl
BOC for tert-butyloxycarbonyl
Trt for trityl
OtBu for tertiary butyl ester, and
tBu for tert-butyl.

### (2) Apparatus and Reagents:

Apparatus: Model 433A peptide synthesizer (Applied Biosystem, US)
Reagents:
N-methyl ketopyrrolidine, methylene chloride, hexahydropyridine, methanol, dimethylaminopyridine/ DMF N, N-diisopropylethylamine/NMP, 100 mmole IIBTU / 0.5 M HOBT in DMF, N, N-Dicyclohexylcarbodiimide/NMP
In which:
DMF stands for N, N-Dimethylformamide
NMP for N-methylpyrrolidone
HOBT for 1-Hydroxybenzotriazole, and
HBTU for 2-(1H-benzotriazole-yl-1,1,3,3-tetramethyl-Uronium hexafluorophosphate)

### (3) Procedures:

### a. Synthesis

Take the synthesis scale 0.25 for example, the synthesis process was described as follows. 0.25g of HMP resin was weighed and placed in a reactor vessel of the synthesizer. 1 mmol of various residues, each coupled with protecting groups, were weighed and arrayed in the synthesizer by the amino acid sequence of the insulinotropic peptide derivate from the carboxy terminal to the amino terminal. At room temperature of 25°C, reactions for removing Fmoc protection, activating a residue and attaching the activated residue to HMP resin were automatically performed under the control of a computer program. Such reactions were circulated until the whole peptide was synthesized. After completion of the synthesis, the residue-attached resin, with each residue coupled with side chain protecting groups, was air dried on a peptide synthesizer and then weighed.

### b. Removal of protecting groups and detachment of resin:

The residue-attached resin, with each residue of the insulinotropic peptide derivate coupled with protecting groups, was placed in a plugged erlenmeyer flask, and followed by addition of cleavage reagents shown as below.

| Reagent | Dosage |
|---|---|
| Water | 0.50 ml |
| methyl phenate | 0.50 ml |
| Phenol | 0.75 g |
| mercaptoethanol | 0.20 ml |
| trifluoroacetic acid | 10.0 ml |
| | |

The electromagnetic stirring reaction was carried out at constant temperature of 30 °C for 6 hours. After filtration step, the aqueous filtrate was collected. The resin was washed with small amount of trifluoroacetic acid. Then the collected aqueous filtrate and the washing solution were mixed together, and ether was added for precipitation. The mixture was filtrated, and the resulted precipitate was washed with small amount of ether. After evaporation in a dehumidifier, the crude product was obtained.

### c. Purification by HPLC and lyophilize

Separation and purification of the crude product was achieved by using preparative HPLC. Final product was obtained after frozen and lyophilized step. Molecular weight of the product was determined using chromatogram-mass spectrogram joint analysis. The peptide derivate was found to have a molecular weigh of 4316.7, the theoretical molecular weigh of which was 4300.6.

### Example 2

### Preparation of an insulinotropic peptide derivate of the present invention wherein X is Leu, Y is Lys and Z is Arg-OH by bio-engineering techniques

### A. Synthesizing gene fragments by the amino acid sequence of the insulinotropic peptide derivate

### B. Cloning

### Ligation:

Two tubes were taken, and fragment (1) and fragment (4) with their OD₂₆₀ₙₘ = 0.1 (optical density at 260nm) were drawn into one tube, while fragment (1) and fragment (4) with the same optical density were drawn into the other tube. Then polynucleotide kinase buffer, polynucleotide kinase and ATPs were added to the two tubes respectively. The reaction mixtures were incubated at 37°C for 60 minutes to make the 5' terminal of the gene fragment phosphorylated. Then the two tubes were placed in a water bath of 95°C and were incubated for 10 minutes. The water bath was stopped to warm up and was naturally cooled down to room temperature, while annealing reaction was carried out during the process. T4 ligase and T4 ligase buffer were added to the two tubes respectively, and the mixtures were incubated overnight at 16°C for ligation of gene fragments.

### Plasmid

A plasmid containing a promotor such as Lac, P_{L} or Tac was drawn in a tube and digested with restriction endonucleases EcoRI and HindIII, The digested plasmid was extracted with hydroxybenzene: chloroform solvent and collected by centrifugation. The aqueous phase was remained and washed three times with chloroform solvent. Centrifugation was continued, and the resulted aqueous phase was precipitated with isopropanol solvent, followed by centrifugation and air-dry step.

The digested plasmid and the ligated fragment were mixed together. T4 ligase and ligase buffer were added to the mixture. Ligation reaction was carried out at room temperature for 3-4 hours.

### Culture of host bacteria cells:

Bacterial cells of E. coli JM103 were incubated with shaking at 37°C in LB liquid medium (1000 ml of LB liquid media containing 10g of peptone, 5g of yeast extracts and 5g of sodium chloride) for 4 hours. After the bacterial cultures were centrifuged, the collected bacterial cells were treated with calcium chloride solution and kept at -4°C for further use.

### Transformation:

The cloned plasmid was transformed to E. coli IM103 host cells. The transformed bacterial cells were incubated in an ice bath for 30 minutes, and then incubated at 42°C for 2 minutes. Bacterial cells were spread on an agar plate containing ampicillin antibiotic, and were incubated overnight at 37 °C. Colony screening was conducted afterwards and those colonies containing recombinant plasmids were kept as positive colonies.

### C. Fermentation:

A screened colony harboring a recombinant plasmid carrying the derivate gene was incubated with shaking in LB liquid medium. 0.5mM of Isopropyl heta-D-Thiogalactopyranoside (IPTG) was added for the purpose of protein induction. Bacterial cells were incubated overnight and harvested by centrifugation. The expressed protein was identified through polyacrylamide gel electrophoresis (PAGE) containing 12% sodium dodecanesulphonate.

### D. Inclusion bodies

Ten bottles, each containing 300ml of bacterial cultures, were incubated with shaking under the conditions mentioned-above. After protein induction step, lysis solution (20mM phosphonic acid buffer containing 1% sodium chloride, pH 7.5) and lysozyme were added. The bacterial cultures were incubation at 30°C for 30 minutes and centrifugation was followed. The collected precipitate was treated with 6M guanidine hydrochloride (Gu·HCl) for extraction of inclusion bodies. Centrifugation was continued and the resulted supernatant was dialyzed to remove guanidine hydrochloride. The dialysate was washed three times with 20mM phosphonic acid buffer (containing 1% sodium chloride and 0.1% Tween 80, pH 7.5), and inclusion bodies were obtained thereafter.

### E. Lysis

The inclusion bodies were dissolved in 8M carbamide solution. Hydrochloric acid was added to a concentration of 50mM. After addition of cyanogen bromide, the solution was stirred under the shunning of light and the protection of nitrogen. Lysis reaction was conducted for 2 hours, followed by HPLC analysis.

### F. Purification

After completion of the lysis reaction, crude product was obtained through an partition chromatography on Sephadex G-25 and final product was acquired through HPLC purification. Similar to the result of solid phase synthesis, it was shown by mass spectrum analysis that the determined molecular weight of the peptide derivate is consistent with the theoretical weight.

### Example 3

### Pharmacodynamic Studies

Experiments were conducted in two-hour-fasted non-obese diabetic mice (NOD mice) weighing 17±2g. Mice in control group 1 received abdominal injection of 2µg insulin, mice in control group 2 received abdominal injection of 4µg insulin, while those in experiment group received abdominal injection of 0.1 µg of the insulinotropic peptide derivate obtained as described in Example 1.

Blood samples were collected at different minutes. Plasma glucose concentrations were determined using a plasma glucose testing kit (Shanghai Institute of Biological Products Ministry of Health). The results have been shown in figures. It can be observed that the decreasing effect of the insulinotropic peptide derivate on blood glucose is quite obvious.

### Example 4

The decreasing effects of Exendin 4(Lys₂₀, Arg₄₀) not accordingly to the present invention and Exendin 4 (Leu₁₄, Lys₂₀, Arg₄₀) of the present invention on blood glucose in NOD mice Materials and Method:

NOD mice were provided by Shanghai Laboratory Animal Center of Chinese Academy of Sciences. 0.9% sodium chloride solution, Exendin 4(Lys₂₀, Arg₄₀) and Exendin 4 (Leu₁₄, Lys₂₀, Arg₄₀) peptides were used in the assay. Plasma glucose testing kit was purchased from Shanghai Institute of Biological Products Ministry of Health.

Overnight-fasted NOD mice were divided to three groups. Mice in the first group were abdominally injected with 200 µl solution containing 40% glucose and 1 µg of Exendin 4(Lys₂₀, Arg₄₀). Mice in the second group were abdominally injected with 200 µl solution containing 40% glucose and 2µg of Exendin 4(Leu₁₄, Lys₂₀, Arg₄₀). While in the third group, also the control group, mice were abdominally injected with glucose solution.

30 µl of blood sample was taken from retro-orbital zeinous sinus immediately using a scaled capillary which was treated beforehand with heparin. The sample was put into 300 µl of normal saline and mixed with the saline. Erythrocyte was removed by centrifugation at 3000 rpm, while blood serum was kept for glucose determination. Different blood samples were taken as described above at 30 min, 60 min and 120 min respectively, then blood serums were separated. Glucose concentrations of three plasma samples were determined according to the method described by the testing kit, and the decreasing effect of GLP-1 (7-36) on blood glucose concentration was detected.

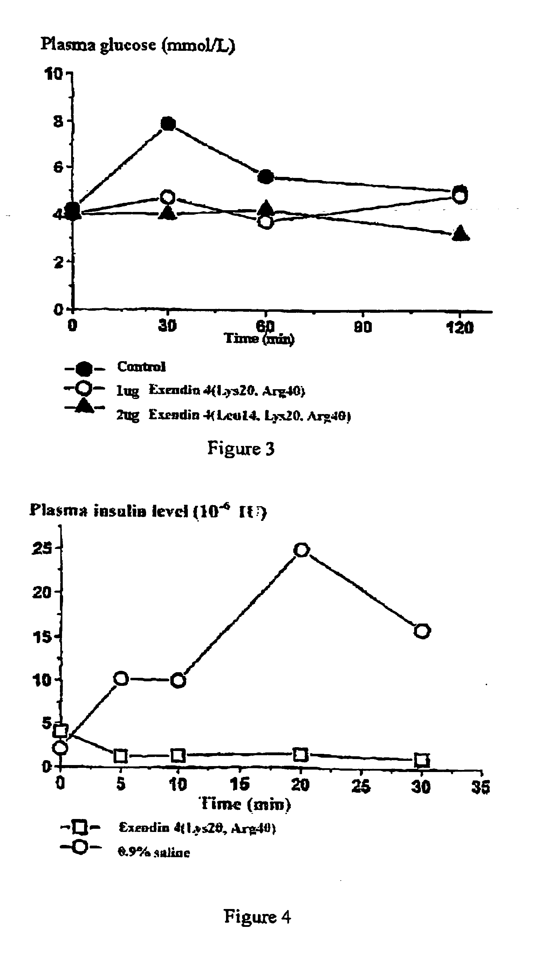

As shown in figure 3, after a dramatic increase, blood glucose concentration in the control group returned to a normal level gradually; while glucose concentrations in the experiment groups never showed notable increase and kept a normal level all the time. The reason is that administration of Exendin 4 derivate stimulates insulin secretion and therefore avoids dramatic fluctuate of the glucose concentration. Thus, the decreasing effects of Exendin 4(Lys₂₀, Arg₄₀) and Exendin 4 (Leu₁₄, Lys₂₀, Arg₄₀) on blood glucose are supported by the experiment results.

### Example 5

### Stimulating effect of Exendin 4(Lys₂₀, Arg₄₀) on insulin secretion

### Materials and Method:

NOD mice were provided by Shanghai Laboratory Animal Center of Chinese Academy of Sciences, 40% glucose solution, 0.9% sodium chloride solution and Exendin 4(Lys₂₀, Arg₄₀) were used in the experiment. Insulin radioimmunoassay kit was purchased from Shanghai Institute of Biological Products Ministry of Heath.

NOD mice were divided into two groups. 50 µl of blood sample was taken from plexus venosus of the eye using scaled capillary, the inner wall of which was rinsed with 1 mg/mL heparin and was air-dried beforehand. Mice in the two groups were abdominally injected with 200 µl normal saline containing 5µg Exendin 4(Lys₂₀, Arg₄₀), respectively, and this moment was recorded as 0 minute. Different blood samples were taken as described above at 5 min, 10 min, 20 min and 30 min respectively. After sampling, each blood sample was immediately put into a centrifuge tube containing 50 [micro]l of normal saline and was mixed with the saline. Erythrocyte was removed by centrifugation at 3000 rpm. Insulin concentrations of different samples were determined by following the methods described by the radioimmunoassay kit, and the stimulating effect of Exendin 4(Lys₂₀, Arg₄₀) on insulin secretion was detected.

As shown in figure 4, abdominal injection with Exendin 4(Lys₂₀, Arg₄₀) can stimulate insulin secretion significantly.

### Example 6

### Stimulating effect of Exendin 4(Lys₂₀, Arg₄₀) on C-peptide secretion

### Materials and Method:

Healthy C₅₇/BL mice were provided by Shanghai Laboratory Animal Center of Chinese Academy of Sciences. 40% glucose solution, 0.9% sodium chloride solution and Exendin 4(Lys₂₀, Arg₄₀) were used in the experiment. Insulin radioimmunoassay kit and C-peptide radioimmunoassay kit were purchased from Shanghai Institute of Biological Products Ministry of Health.

Healthy C₅₇/BL mice were divided into two groups. 50 µl of blood sample was taken from plexus venosus of the eye using scaled capillary, the inner wall of which was rinsed with 1 mg/mL heparin and was air-dried beforehand. Mice in two groups were abdominally injected with 200 µl normal saline containing 5µg of Exendin 4(Lys₂₀, Arg₄₀), respectively, and this moment was recorded as 0 minute. Different blood samples were taken as described above at 5 min, 10 min, 20 min and 30 min respectively. After sampling, each sample was immediately put into a centrifuge tube containing 50 µl of normal saline and was mixed with the saline. Erythrocyte was removed by centrifugation at 3000 rpm. C peptide concentrations of different samples were determined by following the method described by the radioimmunoassay kit, and the stimulating effect of Exendin 4(Lys₂₀, Arg₄₀) on C peptide secretion was detected.

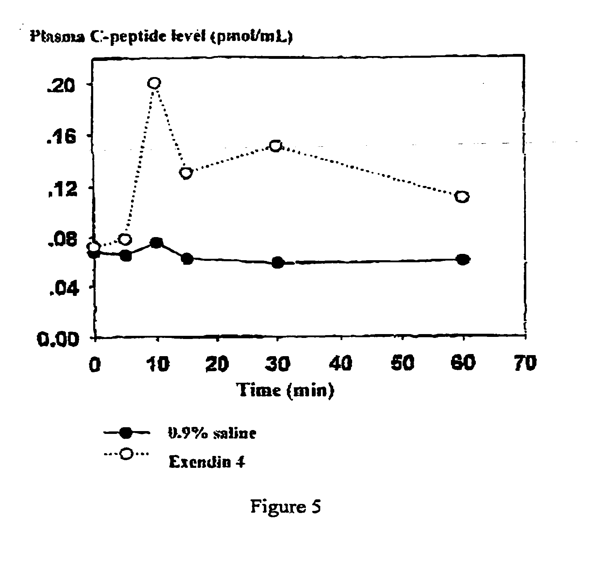

As shown in figure 5, the abdominal injection of Exendin 4(Lys₂₀, Arg₄₀) can stimulate the secretion of C-peptide significantly.

## Claims

1. Derivate of an insulinotropic peptide and pharmaceutically acceptable salts thereof, wherein said derivate having the amino acid sequence: wherein
X is Leu;
Y is Lys;
Z is Arg.

2. Method to produce the insulinotropic peptide derivate as described in claim 1 by recombinant techniques, comprising:
a. synthesizing gene fragments by the amino acid sequence of the insulinotropic peptide derivate,
b. obtaining a cloned bacteria strain after ligation of the gene fragments, construction of a recombinant plasmid, culture of bacterial cells and transformation of the recombinant plasmid,
c. extracting inclusion bodies after fermentation of the bacteria strain and collapse of cell walls,
d. obtaining final product after lysis of inclusion bodies, separation of rough product, purification by HPLC and lyophilization step.

3. Derivate of an insulinotropic peptide and pharmaceutically acceptable salts thereof as described in claim 1 for use in medicine.

4. Derivate of an insulinotropic peptide and pharmaceutically acceptable salts thereof as described in claim 1 for use in the treatment of type II diabetes.

5. Use of the insulinotropic peptide derivate and pharmaceutically acceptable salts thereof as described in claim 1 for the preparation of a medicament for the treatment of type II diabetes.

## Revendications

1. Dérivé d'un peptide insulinotropique et ses sels acceptables au plan pharmaceutique, le dérivé ayant la séquence d'acides aminés suivante : où
X est Leu ;
Y est Lys;
Z est Arg.

2. Procédé de production du dérivé de peptide insulinotropique selon la revendication 1 par des techniques recombinantes, comprenant les étapes suivantes :
a. synthèse de fragments de gènes par la séquence d'acides aminés du dérivé de peptide insulinotropique,
b. obtention d'une douche bactérienne clonée après la ligature des fragments de gènes, construction d'un plasmide recombinant, culture de cellules bactériennes et transformation du plasmide recombinant,
c. extraction de corps d'inclusion après fermentation de la souche bactérienne et affaissement des parois cellulaires,
d. obtention du produit final après la lyse des corps d'inclusion, séparation du produit brut, purification par CLHP et étape de lyophilisation.

3. Dérivé d'un peptide insulinotropique et ses sels acceptables au plan pharmaceutique selon la revendication 1 pour un usage médical.

4. Dérivé d'un peptide insulinotropique et ses sels acceptables au plan pharmaceutique selon la revendication 1 pour une utilisation dans le traitement de diabètes de type II.

5. Utilisation du dérivé de peptide insulinotropique et ses sels acceptables au plan pharmaceutique selon la revendication 1 pour la préparation d'un medicament pour le traitement de diabètes de type II.

## Patentansprüche

1. Derivat eines insulinotropen Peptids sowie pharmazeutisch zulässige Salze, wobei das Derivat folgende Aminosäuresequenz aufweist: wobei
X Leu ist;
Y Lys ist;
Z Arg ist.

2. Verfahren zur Herstellung des Derivats des insulinotropen Peptids nach Anspruch 1 durch Rekombinationstechniken, umfassend:
a. Synthese von Genfragmenten anhand der Aminosäuresequenz des Derivats des insulinotropen Peptids,
b. Gewinnung eines geklonten Bakterienstamms nach Ligation der Genfragmente, Konstruktion eines rekombinanten Plasmids, Anzucht von Bakterienzellen und Transformation des rekombinanten Plasmids,
c. Extraktion von Einschlusskörpern nach Fermentation des Bakterienstamms und Zusammenfall der Zellwände,
d. Gewinnung des Endprodukts nach Lyse der Einschlusskörper, Abtrennung des Rohprodukts, Aufreinigung durch HPLC und Lyophilisationsschritt,

3. Derivat eines insulinotropen Peptids sowie dessen pharmazeutisch zulässige Salze nach Anspruch 1 zur Verwendung in der Medizin.

4. Derivat eines insulinotropen Peptids sowie dessen pharmazeutisch zulässige nach Anspruch 1 zum Verwendung bei der Behandlung von Diabetes Typ II.

5. Verwendung des Derivats des insulinotropen Peptids sowie dessen pharmazeutisch zulässiger Salze nach Anspruch 1 zur Herstellung eines Medikaments zur Behandlung von Diabetes Typ II.


### SEQUENCE LISTING

<110> Shanghai Huayi Bio Lab
<120> insulinotropic Peptide Derivatives
<130> USP 05424286
<150> CN 01112856.9
   <151> 2001-05-10
<160> 4
<170> PalentIn version 3.1
<210> 1
   <211> 40
   <212> PRT
   <213> Exendin 4 Analogue
<220>
   <221> VARIANT
   <222> (14)..(14)<223> Leu or Ile or Met
<220>
   <221> VARIANT
   <222> (20)..(20)<223> Arg or Lys
<220>
   <221> VARIANT<22> (40)..(40)
   <223> Lys
<400> 1
   His Gly Glu Gly Thr Phe Thr Ser Asp Leu Ser Lys Gln Arg Glu Glu
   1 5 10 15
   Glu Ala Val His Leu Phe Ile Glu Trp Leu Lys Asn Gly Gly Pro Ser
   20 25 30
   Ser Gly Ala Pro Pro Pro Ser Arg
   35 40
<210> 2
   <211> 39
   <212> PRT
   <213> Exendin 4 Analogue
<220>
   <221> VARIANT
   <222> (14)..(14)<223> Leu or Ile or Met
<220>
   <221> VARIANT
   <222> (20)..(20)
   <223> Arg or Lys
<400> 2
   His Gly Glu Gly Thr Phe Thr Ser Asp Leu Ser Lys Gln Arg Glu Glu
   1 5 10 15
   Glu Ala Val His Leu Phe Ile Glu Trp Leu Lys Asn Gly Gly Pro Ser
   20 25 30
   Ser Gly Ala Pro Pro Pro Ser
   35
<210> 3
   <211> 39
   <212> PRT
   <213> Exendin 4 Analogue
<220>
   <221> VARIANT
   <222> (14)..(14)
   <223> Leu or Ile
<220>
   <221> VARIANT
   <222> (20)..(20)
   <223> Arg or Lys
<220>
   <221> MOD_RES
   <222> (39)..(39)
   <223> AMIDATION
<400> 3
   His Gly Glu Gly Thr Phe Thr Ser Asp Leu Ser Lys Gln Arg Glu Glu
   1 5 10 15
   Glu Ala Val His Leu Phe Ile Glu Trp Leu Lys Asn Gly Gly Pro Ser
   20 25 30
   Ser Gly Ala Pro Pro Pro Ser
   35
<210> 4
   <211> 39
   <212> PRT
   <213> Exendin 4 Analogue
<220>
   <221> VARIANT
   <222> (20)..(20)
   <223> Lys
<220>
   <221> MOD_RES
   <222> (39)..(39)
   <223> AMIDATION
<400> 4
   His Gly Glu Gly Thr Phe Thr Ser Asp Leu Ser Lys Gln Met Glu Glu
   1 5 10 15
   Glu Ala Val His Leu Phe Ile Glu Trp Leu Lys Asn Gly Gly Pro Ser
   20 25 30
   Ser Gly Ala Pro Pro Pro Ser
   35